# EUROPEAN PATENT APPLICATION

(11) **EP 4 696 244 A1**
(43) Date of publication of application: **18.02.2026**
(21) Application number: 25194741.2
(22) Date of filing: 08.08.2025
(51) Int. Cl.: A61B 8/08, A61B 8/00

(54) **ULTRASOUND DIAGNOSTIC APPARATUS AND CONTROL METHOD FOR ULTRASOUND DIAGNOSTIC APPARATUS**

(30) Priority: 15.08.2024 JP 2024135553
(71) Applicant: FUJIFILM Corporation, Tokyo 106-8620 (JP)
(72) Inventor: EBATA, Tetsurou, Kaisei-machi, 258-8538 (JP)
(74) Representative: Meissner Bolte Partnerschaft mbB

(57) **Abstract**

Provided are an ultrasound diagnostic apparatus that enables a user to easily capture a cross section of an organ suitable for measurement, and a control method for the ultrasound diagnostic apparatus.

An ultrasound diagnostic apparatus includes: an image acquisition unit that acquires a plurality of frames of ultrasound images in which a measurement target organ is imaged by transmitting and receiving an ultrasound beam using an ultrasound probe; a feature value calculation unit that calculates a feature value of the measurement target organ in each of the plurality of frames of ultrasound images by performing image analysis on the plurality of frames of ultrasound images; a probe operation direction calculation unit that calculates a target operation direction of the ultrasound probe for imaging an optimal measurement cross section suitable for measuring the measurement target organ based on a time series change in a plurality of the feature values calculated in the plurality of frames of ultrasound images; and a notification unit that notifies a user of the target operation direction.

## Description

### BACKGROUND OF THE INVENTION

### 1. Field of the Invention

The present invention relates to an ultrasound diagnostic apparatus that images an organ of a subject and a control method for the ultrasound diagnostic apparatus.

### 2. Description of the Related Art

In the related art, measurements of organs within a subject have been made by capturing ultrasound images showing tomograms of the subject using a so-called ultrasound diagnostic apparatus. Generally, since organs within a subject vary from person to person in terms of a shape, a size, a position, and an inclined angle, and the like, in order to image a cross section suitable for measuring the target organ, the position, the inclined angle, and the like of an ultrasound probe need to be adjusted to suit the subject. In particular, in the case of imaging cross sections suitable for measurement of an organ having a complex structure such as the heart, delicate adjustments are required regarding the position, the inclined angle, and the like of the ultrasound probe.

Therefore, in order for a user of the ultrasound diagnostic apparatus to easily image cross sections for appropriately measuring a measurement target organ, for example, an ultrasound diagnostic apparatus such as that disclosed in JP2016-214393A has been developed. The ultrasound diagnostic apparatus disclosed in JP2016-214393A displays, to a user, information regarding a time-series increase or decrease in the major axis diameter of the left ventricle calculated from a plurality of consecutive frames of ultrasound images in which the left ventricle of a subject's heart is imaged. While checking information regarding the time-series increase or decrease in the calculated value of the major axis diameter of the left ventricle, the user adjusts the position, the inclined angle, and the like of the ultrasound probe in order to image a cross section of the left ventricle suitable for measuring the left ventricular ejection fraction (LVEF) of the heart.

### SUMMARY OF THE INVENTION

Even in a case in which the technology disclosed in JP2016-214393A is used to check information regarding the time-series increase or decrease in the calculated value of the major axis diameter of the left ventricle, depending on the user's level of skill, it may be difficult to determine in which direction the ultrasound probe should actually be moved or how the ultrasound probe should be inclined in order to image a cross section suitable for measuring the left ventricle, and the user may not be able to easily image the desired cross section.

The present invention has been made to solve such problems in the related art, and an object of the present invention is to provide an ultrasound diagnostic apparatus that enables a user to easily image a cross section of an organ suitable for measurement and a control method for the ultrasound diagnostic apparatus.

The above object can be achieved by the following configuration.
[1] An ultrasound diagnostic apparatus comprising:
   an ultrasound probe;
   an image acquisition unit that acquires a plurality of frames of ultrasound images in which a measurement target organ of a subject is imaged by transmitting and receiving an ultrasound beam using the ultrasound probe;
   a feature value calculation unit that calculates a feature value of the measurement target organ in each of the plurality of frames of ultrasound images by performing image analysis on the plurality of frames of ultrasound images acquired by the image acquisition unit;
   a probe operation direction calculation unit that calculates a target operation direction of the ultrasound probe for imaging an optimal measurement cross section suitable for measuring the measurement target organ based on a time series change in a plurality of the feature values calculated by the feature value calculation unit in the plurality of frames of ultrasound images; and
   a notification unit that notifies a user of the target operation direction calculated by the probe operation direction calculation unit.
[2] The ultrasound diagnostic apparatus according to [1], in which the probe operation direction calculation unit starts calculating the target operation direction with a trigger of an elapse of a predetermined time after the image acquisition unit starts acquiring the ultrasound image.
[3] The ultrasound diagnostic apparatus according to [1] or [2], further comprising an optimal feature value selection unit that selects one of the plurality of feature values calculated by the feature value calculation unit as an optimal feature value corresponding to the optimal measurement cross section,
   in which the notification unit notifies the user of the optimal feature value selected by the optimal feature value selection unit.
[4] The ultrasound diagnostic apparatus according to [3], in which the notification unit notifies the user to acquire the plurality of frames of ultrasound images while moving the ultrasound probe.
[5] The ultrasound diagnostic apparatus according to [3], further comprising a feature value memory that stores the optimal feature value in association with the subject.
[6] The ultrasound diagnostic apparatus according to any one of [3] to [5], in which the measurement target organ is a heart.
[7] The ultrasound diagnostic apparatus according to [6], in which the feature value calculation unit calculates a depth of an upper end part of a cardiac cavity in the plurality of frames of ultrasound images as the feature value, and
   the probe operation direction calculation unit
   calculates a current movement direction of the ultrasound probe by the user along a body surface of the subject as the target operation direction in a case in which the depth calculated by the feature value calculation unit changes to become shallower in time series, and
   calculates an opposite direction to the current movement direction of the ultrasound probe by the user along the body surface of the subject as the target operation direction in a case in which the depth calculated by the feature value calculation unit changes to become deeper in time series.
[8] The ultrasound diagnostic apparatus according to [6] or [7], in which the feature value calculation unit calculates a length of a major axis of a cardiac cavity as the feature value, and
   the probe operation direction calculation unit
   calculates a current inclination direction of the ultrasound probe by the user as the target operation direction in a case in which the length calculated by the feature value calculation unit changes to become longer in time series, and
   calculates an opposite direction to the current inclination direction of the ultrasound probe by the user as the target operation direction in a case in which the length calculated by the feature value calculation unit changes to become shorter in time series.
[9] The ultrasound diagnostic apparatus according to [6] or [7], in which the feature value calculation unit calculates an area of a cardiac cavity as the feature value, and
   the probe operation direction calculation unit
   calculates a current inclination direction of the ultrasound probe by the user as the target operation direction in a case in which the area calculated by the feature value calculation unit changes to become larger in time series, and
   calculates an opposite direction to the current inclination direction of the ultrasound probe by the user as the target operation direction in a case in which the area calculated by the feature value calculation unit changes to become smaller in time series.
[10] The ultrasound diagnostic apparatus according to any one of [6] to [9], in which the plurality of feature values change in time series due to pulsation of the heart to have a plurality of maximum values and a plurality of minimum values, and
   the probe operation direction calculation unit calculates the target operation direction based on a time series change in the plurality of maximum values or the plurality of minimum values.
[11] The ultrasound diagnostic apparatus according to any one of [6] to [10], in which the image acquisition unit acquires the plurality of frames of ultrasound images in order for each of an apical four-chamber cross section and an apical two-chamber cross section of the heart,
   the feature value calculation unit calculates the plurality of feature values for a first cross section among the apical four-chamber cross section and the apical two-chamber cross section for which the plurality of frames of ultrasound images have been acquired first by the image acquisition unit,
   the optimal feature value selection unit selects the optimal feature value from the plurality of feature values calculated for the first cross section, and
   the notification unit notifies the user of the optimal feature value selected for the first cross section while the plurality of frames of ultrasound images for a second cross section among the apical four-chamber cross section and the apical two-chamber cross section that is different from the first cross section are being acquired by the image acquisition unit.
[12] The ultrasound diagnostic apparatus according to any one of [6] to [11], in which the feature value calculation unit calculates a similarity between the plurality of frames of ultrasound images and a reference image corresponding to the optimal measurement cross section as the feature value, and
   the probe operation direction calculation unit
   calculates a current rotation direction of the ultrasound probe by the user as the target operation direction in a case in which a plurality of the similarities calculated by the feature value calculation unit change to become larger in time series, and
   calculates an opposite direction to the current rotation direction of the ultrasound probe by the user as the target operation direction in a case in which the plurality of similarities calculated by the feature value calculation unit change to become smaller in time series.
[13] The ultrasound diagnostic apparatus according to any one of [1] to [12], further comprising a position and posture sensor that detects a position and an inclined angle of the ultrasound probe,
   in which the probe operation direction calculation unit calculates the target operation direction by further taking into account the position and the inclined angle of the ultrasound probe detected by the position and posture sensor.
[14] The ultrasound diagnostic apparatus according to any one of [1] to [13], further comprising a monitor, in which the notification unit notifies the user of the target operation direction by displaying the target operation direction on the monitor.
[15] A control method for an ultrasound diagnostic apparatus, the control method comprising:
   acquiring a plurality of frames of ultrasound images in which a measurement target organ of a subject is imaged by transmitting and receiving an ultrasound beam using an ultrasound probe;
   calculating a feature value of the measurement target organ in each of the plurality of frames of ultrasound images by performing image analysis on the plurality of frames of ultrasound images that have been acquired;
   calculating a target operation direction of the ultrasound probe for imaging an optimal measurement cross section suitable for measuring the measurement target organ based on a time series change in a plurality of the feature values that have been calculated in the plurality of frames of ultrasound images; and
   notifying a user of the calculated target operation direction.

According to the present invention, an ultrasound diagnostic apparatus comprises: an ultrasound probe; an image acquisition unit that acquires a plurality of frames of ultrasound images in which a measurement target organ of a subject is imaged by transmitting and receiving an ultrasound beam using the ultrasound probe; a feature value calculation unit that calculates a feature value of the measurement target organ in each of the plurality of frames of ultrasound images by performing image analysis on the plurality of frames of ultrasound images acquired by the image acquisition unit; a probe operation direction calculation unit that calculates a target operation direction of the ultrasound probe for imaging an optimal measurement cross section suitable for measuring the measurement target organ based on a time series change in a plurality of the feature values calculated by the feature value calculation unit in the plurality of frames of ultrasound images; and a notification unit that notifies a user of the target operation direction calculated by the probe operation direction calculation unit. Therefore, a user can easily image a cross section of an organ suitable for measurement.

### BRIEF DESCRIPTION OF THE DRAWINGS

Fig. 1 is a block diagram showing a configuration of an ultrasound diagnostic apparatus according to an embodiment of the present invention.
Fig. 2 is a block diagram showing a configuration of a transmission/reception circuit in the embodiment of the present invention.
Fig. 3 is a block diagram showing a configuration of an image generation unit in the embodiment of the present invention.
Fig. 4 is a diagram schematically showing an ultrasound image representing an apical four-chamber cross section of a heart.
Fig. 5 is a diagram schematically showing an optimal measurement cross section suitable for measuring a left ventricular ejection fraction of a heart of a subject.
Fig. 6 is a diagram schematically showing a cross section of the heart in a case in which an ultrasound probe is disposed at a position different from the position corresponding to the optimal measurement cross section.
Fig. 7 is a diagram showing a display example of a target operation direction of an ultrasound probe in the embodiment of the present invention.
Fig. 8 is a flowchart showing an operation of the ultrasound diagnostic apparatus according to the embodiment of the present invention.
Fig. 9 is a graph showing an example of a feature value whose value changes periodically due to pulsation of the heart of the subject.
Fig. 10 is a diagram schematically showing an ultrasound image representing an apical two-chamber cross section of a heart.

### DESCRIPTION OF THE PREFERRED EMBODIMENTS

Hereinafter, embodiments of the present invention will be described based on the accompanying drawings.

The following description of the components will be given based on a representative embodiment of the present invention, but the present invention is not limited to such an embodiment.

In addition, in the present specification, a numerical range represented using "to" means a range including numerical values described before and after "to" as a lower limit value and an upper limit value.

In the present specification, the terms "same" and "identical" include an error range that is generally allowed in the technical field.

### Embodiment

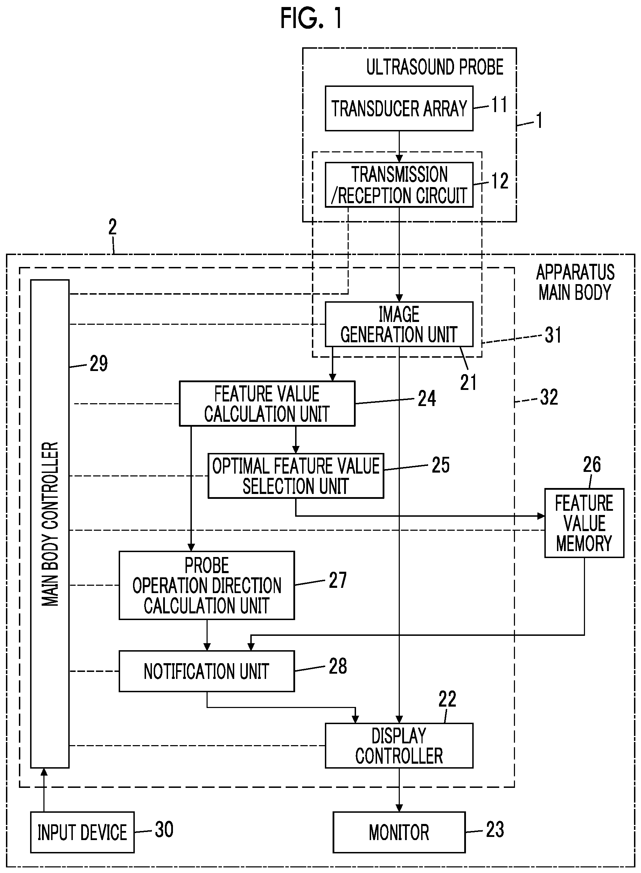

Fig. 1 shows a configuration of an ultrasound diagnostic apparatus according to an embodiment of the present invention. The ultrasound diagnostic apparatus comprises an ultrasound probe 1, and an apparatus main body 2 connected to the ultrasound probe 1. The ultrasound probe 1 and the apparatus main body 2 are connected to each other via so-called wired communication or so-called wireless communication.

The ultrasound probe 1 includes a transducer array 11. A transmission/reception circuit 12 is connected to the transducer array 11.

The apparatus main body 2 includes an image generation unit 21 connected to the transmission/reception circuit 12 of the ultrasound probe 1. A display controller 22 and a monitor 23 are sequentially connected to the image generation unit 21. A feature value calculation unit 24 is connected to the image generation unit 21. An optimal feature value selection unit 25 and a feature value memory 26 are sequentially connected to the feature value calculation unit 24. Further, a probe operation direction calculation unit 27 is connected to the feature value calculation unit 24. A notification unit 28 is connected to the feature value memory 26 and the probe operation direction calculation unit 27. The notification unit 28 is connected to the display controller 22. In addition, a main body controller 29 is connected to the transmission/reception circuit 12, the image generation unit 21, the display controller 22, the feature value calculation unit 24, the optimal feature value selection unit 25, the feature value memory 26, the probe operation direction calculation unit 27, and the notification unit 28. An input device 30 is connected to the main body controller 29.

The transmission/reception circuit 12 and the image generation unit 21 constitute an image acquisition unit 31. In addition, the image generation unit 21, the display controller 22, the feature value calculation unit 24, the optimal feature value selection unit 25, the probe operation direction calculation unit 27, the notification unit 28, and the main body controller 29 constitute a processor 32 for the apparatus main body 2.

The transducer array 11 of the ultrasound probe 1 includes a plurality of ultrasound transducers arranged one-dimensionally or two-dimensionally. In response to a drive signal supplied from the transmission/reception circuit 12, each of the ultrasound transducers transmits ultrasound and receives an ultrasound echo from a subject to output a signal based on the ultrasound echo. For example, each ultrasound transducer is configured by forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 31 configured by the transmission/reception circuit 12 and the image generation unit 21 acquires an ultrasound image of the inside of the subject by transmitting and receiving an ultrasound beam by using the ultrasound probe 1.

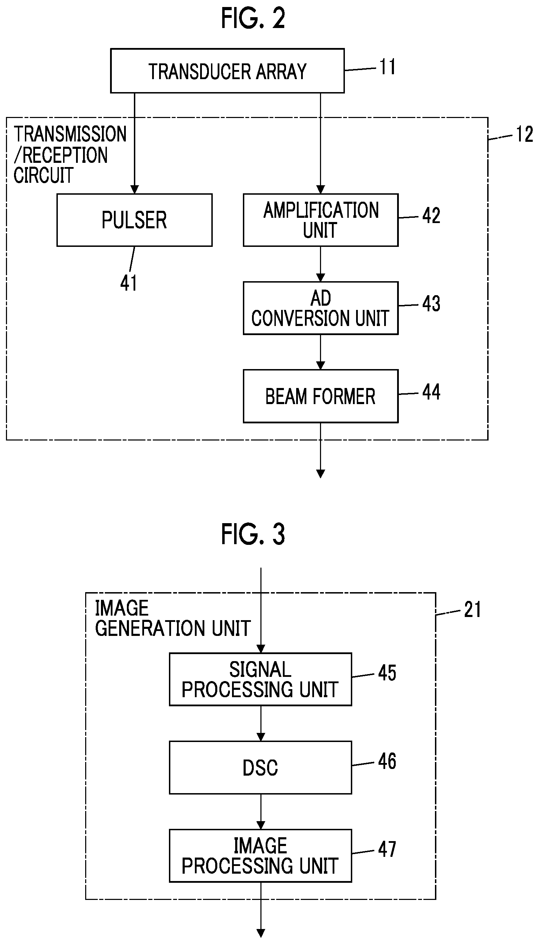

Under the control of the main body controller 29, the transmission/reception circuit 12 causes the transducer array 11 to transmit the ultrasound and generates a sound ray signal based on a reception signal acquired by the transducer array 11. As shown in Fig. 2, the transmission/reception circuit 12 includes a pulser 41 connected to the transducer array 11, and an amplification unit 42, an analog-to-digital (AD) conversion unit 43, and a beam former 44 that are sequentially connected in series to the transducer array 11.

The pulser 41 includes, for example, a plurality of pulse generators, and the pulser 41 adjusts an amount of delay of each drive signal such that the ultrasound transmitted from the plurality of ultrasound transducers of the transducer array 11 form the ultrasound beam, based on a transmission delay pattern selected in accordance with a control signal from the main body controller 29, and supplies the adjusted signal to the plurality of ultrasound transducers. In this way, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, whereby the ultrasound beam is formed from the combined wave of the ultrasound.

The transmitted ultrasound beam is, for example, reflected in a target such as a part of the subject and propagates toward the transducer array 11 of the ultrasound probe 1. The ultrasound echo propagating toward the transducer array 11 in this manner is received by each of the ultrasound transducers constituting the transducer array 11. In this case, each of the ultrasound transducers constituting the transducer array 11 receives the propagating ultrasound echo to expand and contract to generate the reception signal, which is an electric signal, and outputs these reception signals to the amplification unit 42.

The amplification unit 42 amplifies the signal input from each of the ultrasound transducers constituting the transducer array 11 and transmits the amplified signal to the AD conversion unit 43. The AD conversion unit 43 converts the signal transmitted from the amplification unit 42 into digital reception data. The beam former 44 performs so-called reception focusing processing by adding up each piece of reception data received from the AD conversion unit 43 after applying a respective delay thereto. By this reception focusing processing, each piece of reception data converted by the AD conversion unit 43 is phase-added, and the sound ray signal in which the focus of the ultrasound echo is narrowed down is acquired.

As shown in Fig. 3, the image generation unit 21 has a configuration in which a signal processing unit 45, a digital scan converter (DSC) 46, and an image processing unit 47 are sequentially connected in series to each other.

The signal processing unit 45 generates a B-mode image signal, which is tomographic image information related to tissues inside the subject, by performing, on the sound ray signal received from the transmission/reception circuit 12, correction of the attenuation due to a distance in accordance with a depth of a reflection position of the ultrasound by using a sound velocity value set by the main body controller 29 and then performing envelope detection processing.

The DSC 46 converts (raster-converts) the B-mode image signal generated by the signal processing unit 45 into an image signal conforming to the scanning method of a normal television signal.

The image processing unit 47 performs various types of necessary image processing such as gradation processing on the B-mode image signal input from the DSC 46, and then sends the B-mode image signal to the display controller 22 and the optimal feature value selection unit 25. Hereinafter, the B-mode image signal that has been subjected to image processing by the image processing unit 47 will be referred to as an ultrasound image.

The feature value calculation unit 24 performs image analysis on a plurality of frames of ultrasound images acquired by the image acquisition unit 31 to calculate a feature value of a measurement target organ of a subject in each of the plurality of frames of ultrasound images. The measurement target organ of a subject is an organ within the subject that is the target of a specific measurement. For example, the specific measurement can be set to be the measurement of so-called left ventricular ejection fraction (LVEF), and the measurement target organ can be set to be the heart, which is the target of measurement of the left ventricular ejection fraction. The left ventricular ejection fraction is usually calculated as a ratio of the left ventricular volume at end systole to the left ventricular volume at end diastole.

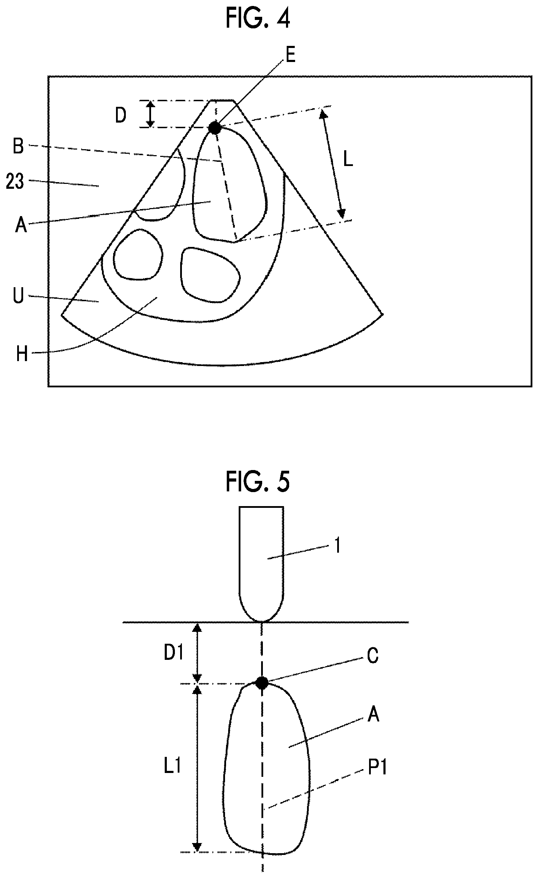

The feature value of the measurement target organ is a measurement value related to the cross sectional structure of the measurement target organ, which is used as an index value for determining whether or not the captured ultrasound image represents the optimal measurement cross section, that is, a cross section suitable for performing a specific measurement of the measurement target organ. For example, in a case of determining the optimal measurement cross section for measuring the left ventricular ejection fraction of a heart H as shown in Fig. 4, a depth D of an upper end part E of a lumen of a left ventricle A in an ultrasound image U and a length of a major axis B of the lumen of the left ventricle A in the ultrasound image U, that is, a major axis diameter L, can be set as feature values. The upper end part E of the lumen of the left ventricle A in the ultrasound image U refers to the shallowest part of the boundary of the lumen of the left ventricle A shown in the ultrasound image U. The major axis B of the lumen of the left ventricle A in the ultrasound image U can be defined as the longest line segment among line segments connecting the upper end part E of the lumen of the left ventricle A to any other position on the boundary of the lumen of the left ventricle A.

In a case of calculating the depth D of the upper end part E of the left ventricle A as a feature value, the feature value calculation unit 24 can extract the contour of the lumen of the left ventricle A from the ultrasound image U through a so-called segmentation method using, for example, a trained model in machine learning that has learned a relationship between the ultrasound image U showing the left ventricle A and the contour of the lumen of the left ventricle A shown therein, and specify the shallowest position among the extracted contours or the position on the extracted contour where the curvature is maximum as the position of the upper end part E. The feature value calculation unit 24 can calculate the depth D by measuring a distance between the specified position of the upper end part E and the upper end part of the ultrasound image U.

In addition, the feature value calculation unit 24 can also extract the upper end part E of the lumen of the left ventricle A and calculate its depth D using an algorithm for extracting characteristic points, that is, so-called key points, in an image, such as scale invariant feature transform (SIFT) and support vector machine (SVM). In this case, the feature value calculation unit 24 extracts the position of the roots of a pair of mitral valves at the lower end part of the left ventricle A using, for example, the algorithm described above, specifies a line segment connecting the midpoint of the extracted roots of the pair of mitral valves and the upper end part E as the major axis B of the lumen of the left ventricle A, and measures the length of the specified major axis B to calculate the major axis diameter L.

In this way, the feature value calculation unit 24 can perform image analysis on the ultrasound image using a segmentation method based on machine learning or an algorithm for extracting key points in the ultrasound image U, and calculate the feature values.

Fig. 5 schematically shows an ideal positional relationship between the lumen of the left ventricle A and the ultrasound probe 1, which allows imaging of an optimal measurement cross section for measuring the left ventricular ejection fraction. In this diagram, the ultrasound probe 1 and the lumen of the left ventricle A are viewed from a direction along a scan plane P1 of the ultrasound probe 1, and the scan plane P1 extends in a direction orthogonal to the paper surface.

In a case in which the ultrasound probe 1 is disposed on the body surface of the subject at an ideal position and inclined angle for imaging an optimal measurement cross section for the left ventricular ejection fraction, the scan plane P1 of the ultrasound probe 1 passes through the apex of the heart C of the left ventricle A, and a major axis diameter L1 of the lumen of the left ventricle A in the ultrasound image U corresponding to the scan plane P1 is longer than in a case in which the ultrasound probe 1 is at any other position and inclined angle. The apex of the heart C corresponds to the upper end part E of the lumen of the left ventricle A, which has the shallowest depth D1 in the ultrasound image U.

Therefore, for example, in a plurality of ultrasound images U acquired corresponding to a plurality of positions and inclined angles of the ultrasound probe 1, in a case in which a plurality of depths D of the upper end part E of the lumen of the left ventricle A and a plurality of major axis diameters L of the lumen of the left ventricle A are calculated as feature values, the minimum depth D1 and the maximum major axis diameter L1 can be determined as optimal feature values corresponding to the optimal measurement cross section for the left ventricular ejection fraction.

The optimal feature value selection unit 25 selects one of the plurality of feature values calculated by the feature value calculation unit 24 as the optimal feature value corresponding to the optimal measurement cross section, based on such criteria, for example. In a case in which the feature value calculation unit 24 sequentially calculates the feature values and calculates a more preferable feature value corresponding to the optimal measurement cross section, for example, in a case in which a smaller value of the depth D or a larger value of the major axis diameter L is calculated, the optimal feature value selection unit 25 updates the optimal feature value by newly selecting the calculated more preferable feature value as the optimal feature value. The optimal feature value selection unit 25 sends the optimal feature value selected in this manner to the feature value memory 26.

The feature value memory 26 is a memory that stores the optimal feature value selected by the optimal feature value selection unit 25. Since it is expected that the values of the depth D1 of the apex of the heart C of the lumen of the left ventricle A and the maximum major axis diameter L1 of the lumen of the left ventricle A will not vary significantly from test to test for the same subject, the feature value memory 26 can store the optimal feature value in association with the subject, for example by associating the optimal feature value with an identifier (ID) of the subject.

Here, as the feature value memory 26, for example, a recording medium such as a flash memory, a hard disk drive (HDD), a solid-state drive (SSD), a flexible disk (FD), a magnetooptical disk (MO disk), a magnetic tape (MT), a random-access memory (RAM), a compact disc (CD), a digital versatile disc (DVD), a secure digital card (SD card), or a universal serial bus memory (USB memory) can be used.

The probe operation direction calculation unit 27 calculates a target operation direction of the ultrasound probe 1 for imaging an optimal measurement cross section suitable for measuring the measurement target organ, based on the time series changes in the plurality of feature values calculated by the feature value calculation unit 24 in the plurality of frames of ultrasound images U. The target operation direction of the ultrasound probe 1 is the direction in which the ultrasound probe 1 should be operated in order to image the optimal measurement cross section, and includes the direction in which the ultrasound probe 1 is moved in parallel along the body surface of the subject, the inclination direction of the ultrasound probe 1, and the like.

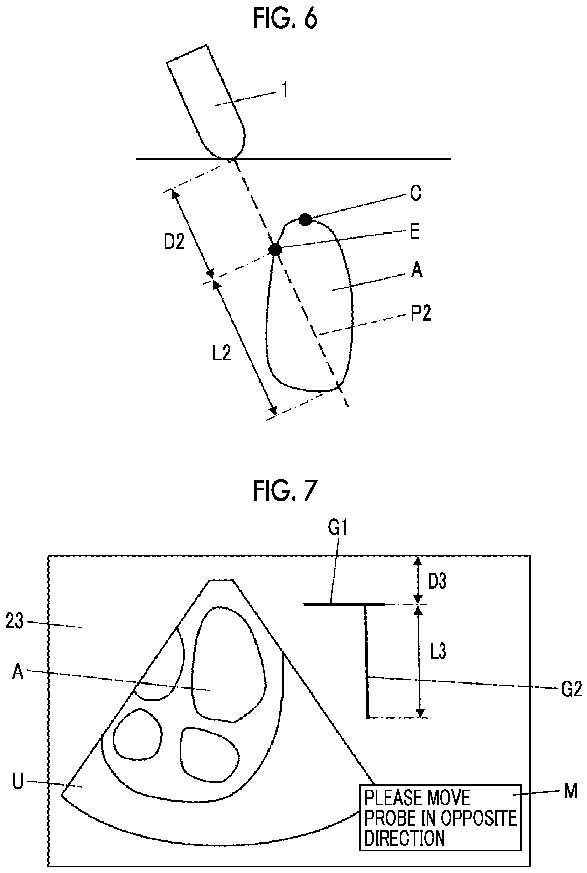

Fig. 6 schematically shows a positional relationship between the ultrasound probe 1 and the lumen of the left ventricle A in a case in which the ultrasound probe 1 is disposed away from the ideal position for imaging an optimal measurement cross section for measuring the left ventricular ejection fraction. In this diagram, similarly to Fig. 5, the ultrasound probe 1 and the lumen of the left ventricle A are viewed from the direction along the scan plane P2 of the ultrasound probe 1.

In a case in which the ultrasound probe 1 is disposed away from the ideal position for imaging the optimal measurement cross section for measuring the left ventricular ejection fraction, even though the inclined angle of the ultrasound probe 1 is adjusted to image a cross section in which the major axis diameter L2 of the lumen of the left ventricle A in the ultrasound image U is maximum, unless the scan plane P2 of the ultrasound probe 1 passes through the apex of the heart C of the lumen of the left ventricle A, the major axis diameter L1 of the lumen of the left ventricle A in the optimal measurement cross section is not obtained. In order for the scan plane P2 to pass through the apex of the heart C and for the major axis diameter L2 of the lumen of the left ventricle A to become maximum, it is necessary to move the ultrasound probe 1 in parallel along the body surface of the subject. In a case in which the ultrasound probe 1 is moved in parallel along the body surface of the subject toward the ideal position, the value of a depth D2 of the upper end part E of the lumen of the left ventricle A changes to gradually decrease in time series, and in a case in which the ultrasound probe 1 is moved in parallel along the body surface of the subject away from the ideal position, the value of the depth D2 of the upper end part E of the lumen of the left ventricle A changes to gradually increase in time series.

Therefore, the probe operation direction calculation unit 27 can calculate the current (parallel) movement direction of the ultrasound probe 1 along the body surface of the subject as the target operation direction in a case in which the value of the depth D2 of the upper end part E of the lumen of the left ventricle A changes to gradually decrease in time series from any time point in the past to the current time point. In addition, the probe operation direction calculation unit 27 can calculate the opposite direction to the current (parallel) movement direction of the ultrasound probe 1 along the body surface of the subject as the target operation direction in a case in which the value of the depth D2 of the upper end part E of the lumen of the left ventricle A changes to gradually increase in time series from any time point in the past to the current time point.

Furthermore, for example, in a case of attempting to obtain the maximum major axis diameter L2 of the lumen of the left ventricle A while keeping the position of the ultrasound probe 1 on the body surface of the subject fixed, it is necessary to adjust the inclined angle of the ultrasound probe 1. Therefore, the probe operation direction calculation unit 27 can calculate the current inclination direction of the ultrasound probe 1 as the target operation direction in a case in which the value of the major axis diameter L2 of the lumen of the left ventricle A changes to gradually increase in time series from any time point in the past to the current time point. In addition, the probe operation direction calculation unit 27 can calculate the opposite direction to the current inclination direction of the ultrasound probe 1 as the target operation direction in a case in which the value of the major axis diameter L2 of the lumen of the left ventricle A changes to gradually decrease in time series from any time point in the past to the current time point.

In order to accurately determine the time series changes in the plurality of feature values, the probe operation direction calculation unit 27 can start calculating the target operation direction with a trigger of an elapse of a predetermined time after the image acquisition unit 31 starts acquiring the ultrasound image U, or acquisition of a predetermined number of frames of the ultrasound image U by the image acquisition unit 31.

The notification unit 28 notifies a user of the ultrasound diagnostic apparatus of the target operation direction calculated by the probe operation direction calculation unit 27 and the optimal feature value selected by the optimal feature value selection unit 25 and stored in the feature value memory 26. The notification unit 28 can display a message M on the monitor 23 to notify the user of the target operation direction, as shown in Fig. 7. **In** the example of Fig. 7, a message M stating "Please move the probe in the opposite direction" is displayed on the monitor 23, indicating that the ultrasound probe 1 is to be moved in parallel to the opposite direction to the current movement direction along the body surface of the subject. Although not shown, the notification unit 28 can also display, on the monitor 23, a message M stating, for example, "Please incline the probe in the opposite direction," indicating that the ultrasound probe 1 should be inclined in the opposite direction to the current inclination direction.

**In** addition, the notification unit 28 can display, on the monitor 23, for example, as optimal feature values, a depth indication line G1 representing a depth D3 of the upper end part E of the lumen of the left ventricle A which has the minimum value among the calculated values, and a major axis indication line G2 indicating the depth position of the major axis B of the lumen of the left ventricle A which has the maximum major axis diameter L3 among the calculated values. In addition, the notification unit 28 can also incline the major axis indication line G2 in accordance with the inclination of the major axis B.

By checking the target operation direction notified by the notification unit 28, the user can easily image the optimal measurement cross section by moving and inclining the ultrasound probe 1 in parallel toward the ideal position and the ideal inclined angle of the ultrasound probe 1 for imaging the optimal measurement cross section. Furthermore, by checking the optimal feature value notified by the notification unit 28, the user can easily determine whether or not the current ultrasound image U displayed on the monitor 23 represents the optimal measurement cross section.

Under the control of the main body controller 29, the display controller 22 performs predetermined processing on the ultrasound image U sent from the image generation unit 21, information indicating the content of the notification to the user by the notification unit 28, and the like, and displays them on the monitor 23.

The monitor 23 is a monitor for displaying the ultrasound image U, the instruction to the user, and the like under the control of the display controller 22, and includes a display device such as a liquid-crystal display (LCD), or an organic electroluminescence (EL) display.

The main body controller 29 controls each unit of the apparatus main body 2 and the transmission/reception circuit 12 of the ultrasound probe 1 on the basis of a control program and the like stored in advance.

The input device 30 is an input device for the user to perform an input operation, and is configured by, for example, a device such as a keyboard, a mouse, a trackball, a touchpad, and a touch sensor disposed in a state of being superimposed on the monitor 23.

The processor 32 including the image generation unit 21, the display controller 22, the feature value calculation unit 24, the optimal feature value selection unit 25, the probe operation direction calculation unit 27, the notification unit 28, and the main body controller 29 may be configured by one or more pieces of hardware, and the type of hardware is not limited. For example, the processor can be configured by hardware such as a central processing unit (CPU), a micro processing unit (MPU), a programmable logic device such as a field-programmable gate array (FPGA), a dedicated circuit for executing specific processing such as an application-specific integrated circuit (ASIC), a graphic processing unit (GPU), or a neural processing unit (NPU). The processor also has various units or means for executing various processes in the present embodiment. Additionally, the types of hardware may be a combination of different types of hardware. In a case in which a plurality of pieces of hardware are configured to execute one or more processes of a certain processor, the plurality of pieces of hardware may be present in devices physically separate from each other, or may be present in the same device. In addition, in any of the embodiments, the order of the processes performed by the processor is not limited to the order described above, and may be changed as appropriate. The hardware is configured by electrical circuits (circuitry) combining circuit elements such as semiconductor elements.

Further, the present embodiment may be implemented by hardware, software, firmware, microcode, or a combination thereof. The software, firmware, and microcode are configured by programs. The program may also be, for example, a group of program modules, each function of which may be implemented by a processor configured to execute each function. The program may be a program code or a number of code segments stored in one or more non-transitory computer-readable media (for example, storage media or other storages). The program may be stored in a plurality of non-transitory computer-readable media that are present in apparatuses physically separate from each other in a divided manner. A program code or a code segment can represent a procedure, a function, a subprogram, a routine, a subroutine, a module, a software package, a class, or any combination of instructions, data structures, or program statements. A program code or a code segment may be connected to another code segment or a hardware circuit by transmitting and receiving information, data, arguments, parameters, or memory content.

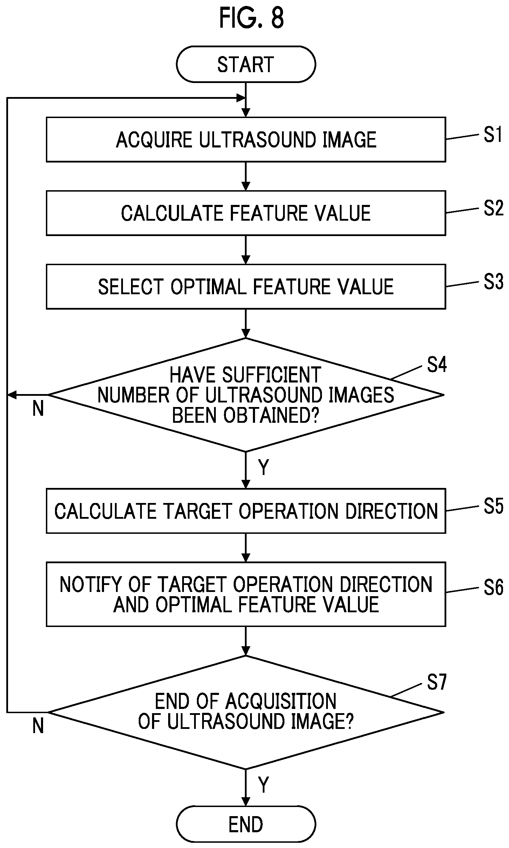

Next, an operation of the ultrasound diagnostic apparatus according to the embodiment will be described with reference to the flowchart shown in Fig. 8.

In Step S1, the image acquisition unit 31 acquires the ultrasound image U obtained by imaging the measurement target organ. In this case, under the control of the main body controller 29, the transmission and reception of the ultrasound from the plurality of transducers of the transducer array 11 are started in accordance with the drive signal from the pulser 41 of the transmission/reception circuit 12 of the ultrasound probe 1, the ultrasound echo from the subject is received by the plurality of transducers of the transducer array 11, and the reception signal as the analog signal is output to the amplification unit 42, is amplified, and then is subjected to the AD conversion via the AD conversion unit 43 to acquire the reception data.

The reception focusing processing is performed on the reception data by the beam former 44, and the sound ray signal generated by the reception focusing processing is sent to the image generation unit 21 of the apparatus main body 2, thereby the ultrasound image U representing the tomographic image information of the subject is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing such as gradation processing. The ultrasound image U thus generated in Step S2 is displayed on the monitor 23 via the display controller 22 and is also sent to the feature value calculation unit 24.

In Step S2, the feature value calculation unit 24 calculates the feature value of the measurement target organ of the subject by performing image analysis on the ultrasound image U acquired in Step S1. The feature value calculation unit 24 can calculate the depth D of the upper end part E of the lumen of the left ventricle A and the major axis diameter L of the lumen of the left ventricle A in the ultrasound image U as feature values of the left ventricle A, which is the target for measuring the left ventricular ejection fraction of the heart H of the subject, for example, by using a segmentation method using machine learning or an algorithm for extracting key points in the ultrasound image U.

In Step S3, the optimal feature value selection unit 25 selects, from the feature values calculated in Step S2, the feature value that is most preferable as the feature value that corresponds to the optimal measurement cross section of the measurement target organ, as the optimal feature value. At the current point in time, since only one feature value for each type of feature value has been calculated in Step S2, the optimal feature value selection unit 25 selects the feature value calculated in Step S2 as the optimal feature value. The feature value memory 26 stores the optimal feature value selected by the optimal feature value selection unit 25 in this manner.

In Step S4, the probe operation direction calculation unit 27 determines whether a sufficient number of feature values have been obtained to calculate a target operation direction of the ultrasound probe 1, that is, whether a sufficient number of ultrasound images U have been obtained. The probe operation direction calculation unit 27 can determine that a sufficient number of ultrasound images U have been obtained in a case in which, for example, a predetermined time, such as 5 seconds, has elapsed since the ultrasound image U was acquired, or in a case in which a predetermined number of frames or more of the ultrasound image U corresponding to that time have been acquired. In addition, the probe operation direction calculation unit 27 can determine that a sufficient number of ultrasound images U have not been obtained in a case in which a predetermined time, such as 5 seconds, has not elapsed since the ultrasound image U was acquired, or in a case in which a predetermined number of frames of ultrasound image U have not been acquired.

In a case in which it is determined in Step S4 that a sufficient number of ultrasound images U have not been obtained, the process returns to Step S1, and a new frame of ultrasound image U is acquired. In the following Step S2, the feature value of the measurement target organ in the ultrasound image U acquired in the immediately preceding Step S1 is calculated.

In Step S3, the optimal feature value selection unit 25 selects one of the feature value calculated in Step S2 for the first time and selected as the optimal feature value and the feature value calculated in Step S2 for the second time as the optimal feature value. For example, in a case in which the depth D of the upper end part E of the lumen of the left ventricle A is calculated as a feature value, the optimal feature value selection unit 25 selects the shallower depth D between the depth D calculated in Step S2 for the first time and the depth D calculated in Step S2 for the second time as the optimal feature value.

In a case in which the feature value calculated in Step S2 for the first time is selected as the optimal feature value, the feature value memory 26 maintains the optimal feature value already stored, and in a case in which the feature value calculated in Step S2 for the second time is selected as the optimal feature value, the feature value memory 26 updates the stored optimal feature value to the feature value calculated in Step S2 for the second time.

In this manner, as long as it is determined in Step S4 that a sufficient number of ultrasound images U have not been obtained, the processes of Steps S1 to S4 are repeated to calculate a plurality of feature values from a plurality of frames of ultrasound images U, and the most preferable feature value among the plurality of calculated feature values as feature value corresponding to the optimal measurement cross section is selected as the optimal feature value and stored in the feature value memory 26.

In a case in which it is determined in Step S4 that a sufficient number of ultrasound images U have been obtained, the process proceeds to Step S5. In Step S5, the probe operation direction calculation unit 27 calculates a target operation direction of the ultrasound probe 1 for imaging an optimal measurement cross section, based on the time series changes in the plurality of feature values calculated by repeating Steps S1 to S4.

For example, the probe operation direction calculation unit 27 can calculate the current movement direction of the ultrasound probe 1 along the body surface of the subject as the target operation direction in a case in which the value of the depth D of the upper end part E of the lumen of the left ventricle A calculated as a feature value changes to gradually decrease from any time point in the past to the present, and the probe operation direction calculation unit 27 can calculate the opposite direction to the current movement direction of the ultrasound probe 1 along the body surface of the subject as the target operation direction in a case in which the value of the depth D of the upper end part E of the lumen of the left ventricle A changes to gradually increase from any time point in the past to the present. In addition, the probe operation direction calculation unit 27 can calculate the current inclination direction of the ultrasound probe 1 as the target operation direction in a case in which, for example, the value of the major axis diameter L of the lumen of the left ventricle A calculated as a feature value changes to gradually increase from any time point in the past to the present, and the probe operation direction calculation unit 27 can calculate the opposite direction to the current inclination direction of the ultrasound probe 1 as the target operation direction in a case in which the value of the major axis diameter L of the lumen of the left ventricle A changes to gradually decrease from any time point in the past to the present.

In Step S6, the notification unit 28 notifies the user of the target operation direction calculated in Step S5 and the optimal feature value finally selected in Step S3 and stored in the feature value memory 26. As shown in Fig. 7, for example, the notification unit 28 can notify the user of the target operation direction and the optimal feature value by displaying, on the monitor 23, a message M indicating the target operation direction, a depth indication line G1 indicating the depth D3 having the minimum value as the optimal feature value, and a major axis indication line G2 indicating the depth position of the major axis B having the maximum major axis diameter L3 as the optimal feature value. The notification unit 28 can also incline the major axis indication line G2 in accordance with the inclination of the major axis B.

**In** Step S7, the main body controller 29 determines whether or not to end acquisition of the ultrasound image U. The main body controller 29 can determine, for example, that the user has been able to image the optimal measurement cross section, and determine to end the acquisition of the ultrasound image U in a case in which the user inputs an instruction to end the acquisition of the ultrasound image U via the input device 30. In addition, the main body controller 29 can determine, for example, that the user has not yet been able to image the optimal measurement cross section, and determine to continue acquiring the ultrasound image U in a case in which no instruction to end the acquisition of the ultrasound image U is specifically input via the input device 30.

As long as it is determined in Step S7 that acquisition of ultrasound images U is continued, the processes in Steps S1 to S7 are repeated. By checking the target operation direction that is sequentially notified in Step S7, the user can easily image the optimal measurement cross section by moving and inclining the ultrasound probe 1 in parallel toward the ideal position and the ideal inclined angle of the ultrasound probe 1 for imaging the optimal measurement cross section. Furthermore, by checking the optimal feature value that is notified in Step S7, the user can easily determine whether or not the current ultrasound image U displayed on the monitor 23 represents the optimal measurement cross section.

In a case in which it is determined in Step S7 that the acquisition of the ultrasound image U is to be ended, the operation of the ultrasound diagnostic apparatus shown in the flowchart of Fig. 8 ends. The user uses the ultrasound image U representing the optimal measurement cross section obtained by this operation to perform measurements of the measurement target organ, such as the left ventricular ejection fraction of the heart H of the subject.

As described above, with the ultrasound diagnostic apparatus according to the embodiment of the present invention, the probe operation direction calculation unit 27 calculates a target operation direction of the ultrasound probe 1 for imaging an optimal measurement cross section based on time-series changes in the plurality of feature values calculated by the feature value calculation unit 24 in the plurality of frames of ultrasound images U, and the notification unit 28 notifies the user of the calculated target operation direction. Therefore, the user can easily image the optimal measurement cross section of the measurement target organ.

Although the transmission/reception circuit 12 has been described as being provided in the ultrasound probe 1, the transmission/reception circuit 12 may be provided in the apparatus main body 2.

In addition, although the image generation unit 21 has been described as being provided in the apparatus main body 2, the image generation unit 21 may be provided in the ultrasound probe 1.

In addition, the apparatus main body 2 may be a so-called stationary type, a portable type that is easy to carry, or a so-called handheld type that is configured by, for example, a smartphone or a tablet type computer. As described above, the type of the device constituting the apparatus main body 2 is not particularly limited.

The notification unit 28 can notify the user that a plurality of frames of ultrasound images U will be acquired while moving the ultrasound probe 1 to image a wide range in order to accurately acquire preferred feature values that correspond to the optimal measurement cross section. By the user moving the ultrasound probe 1 in accordance with this notification, a plurality of frames of ultrasound images U corresponding to various positions and inclined angles of the ultrasound probe 1 can be acquired, thereby making it easy to acquire feature values corresponding to the optimal measurement cross section.

Although it has been described that the notification unit 28 notifies the user by displaying the message M or the like on the monitor 23, the notification method by the notification unit 28 is not limited to the above-described method. For example, in a case in which the ultrasound diagnostic apparatus is equipped with a speaker (not shown), the notification unit 28 can notify the user by emitting voice, warning sound, or the like from the speaker. Furthermore, for example, in a case in which the ultrasound diagnostic apparatus is equipped with a lamp (not shown), the blinking pattern of the lamp can be changed to notify the user. Furthermore, for example, in a case in which the ultrasound probe 1 comprises a vibration device such as a so-called small vibration motor, the notification unit 28 can notify the user by vibrating the ultrasound probe 1, for example.

It is described that the user can determine whether or not the current ultrasound image U displayed on the monitor 23 represents the optimal measurement cross section by checking the optimal feature value notified by the notification unit 28, but the main body controller 29 can, for example, automatically determine this. In this case, the main body controller 29 can determine that the current ultrasound image U represents the optimal measurement cross section, for example, in a case in which the value of the optimal feature value stored in the feature value memory 26 has not been updated for a certain period of time or more, and the difference between the feature value calculated for the current ultrasound image U displayed on the monitor 23 and the optimal feature value is within a certain range. For example, in a case in which the depth D of the upper end part E of the lumen of the left ventricle A and the major axis diameter L of the lumen of the left ventricle A are calculated as feature values, the main body controller 29 can determine that the current ultrasound image U represents the optimal measurement cross section in a case in which both the difference between the depth D and its optimal feature value, and the difference between the major axis diameter L and its optimal feature value are within certain ranges.

Furthermore, in this case, the notification unit 28 can notify the user that the optimal measurement cross section has been imaged by displaying, for example, a message M on the monitor 23. Accordingly, even a user with a low level of skill can easily ascertain that the optimal measurement cross section has been imaged.

Here, in order to determine whether an ultrasound image U representing the optimal measurement cross section for calculating the left ventricular ejection fraction has been acquired, it is described that a determination is made as to whether both the depth D of the upper end part E of the lumen of the left ventricle A and the major axis diameter L of the lumen of the left ventricle A substantially match the corresponding optimal feature values. In this way, in order to determine whether an ultrasound image U representing the optimal measurement cross section for a specific measurement has been acquired, it is necessary to determine whether one or more types of feature values defined corresponding to the optimal measurement cross section substantially match the respective optimal feature values.

Therefore, the main body controller 29 can specify the feature value in question, that is, the feature value that does not substantially match the optimal feature value, by determining, for example, whether one or more types of feature values defined corresponding to the optimal measurement cross section substantially match the respective optimal feature values, that is, whether the difference is within a certain range. In this case, the notification unit 28 can notify the user of the feature value in question. The notification unit 28 can display a message M such as "The apex of the heart cannot be correctly depicted" on the monitor 23, for example, in a case in which the depth D of the upper end part E of the lumen of the left ventricle A does not substantially match the depth D1 of the apex of the heart C. In addition, the notification unit 28 can display a message M such as "The maximum diameter surface cannot be depicted" on the monitor 23, for example, in a case in which the major axis diameter L of the lumen of the left ventricle A does not substantially match the maximum major axis diameter L1. Accordingly, the user can easily ascertain the target for imaging an optimal measurement cross section and move the ultrasound probe 1 appropriately.

Although it has been described that the feature value calculation unit 24 calculates the major axis diameter L of the lumen of the left ventricle A as a feature value, it is also possible to calculate the area of the lumen of the left ventricle A in the ultrasound image U instead of the major axis diameter L of the lumen of the left ventricle A. In this case, the probe operation direction calculation unit 27 can calculate the current inclination direction of the ultrasound probe 1 as the target operation direction in a case in which the value of the area of the lumen of the left ventricle A changes to gradually increase from any time point in the past to the present, and the probe operation direction calculation unit 27 can calculate the opposite direction to the current inclination direction of the ultrasound probe 1 as the target operation direction in a case in which the value of the area of the lumen of the left ventricle A changes to gradually decrease from any time point in the past to the present.

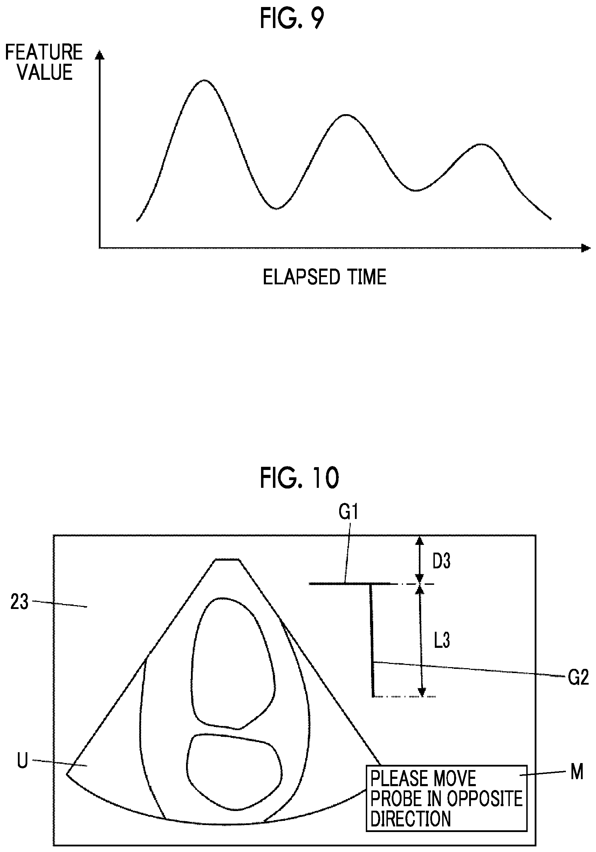

The feature values represented by the depth D of the upper end part E of the lumen of the left ventricle A, the major axis diameter L of the lumen of the left ventricle A, the area of the lumen of the left ventricle A, and the like change in a time series with the pulsation of the heart H to have a plurality of maximum values and a plurality of minimum values, as shown in Fig. 9. In this diagram, three maximum values and two minimum values are shown as an example. In order to accurately obtain the time series changes in these feature values due to the position or the inclined angle of the ultrasound probe 1, the probe operation direction calculation unit 27 can calculate a target operation direction based on the time series changes in a plurality of maximum values of these feature values or the time series changes in a plurality of minimum values of these feature values.

Known cross sections of the heart H imaged by an ultrasound diagnostic apparatus include a so-called apical four-chamber cross section including the left ventricle A, a right ventricle, a left atrium, and a right atrium as shown in Figs. 4 and 7, and a so-called apical two-chamber cross section including the left ventricle A and the left atrium or including the right atrium and the right ventricle as shown in Fig. 10. In a case in which the ultrasound probe 1 is rotated by 90 degrees about a direction in which the user presses the ultrasound probe 1 against the body surface of the subject from a state in which one of the apical four-chamber cross section and the apical two-chamber cross section is imaged, the other of the apical four-chamber cross section and the apical two-chamber cross section can be imaged.

Therefore, in order to determine the appropriate rotation direction of the ultrasound probe 1 in a case in which the apical four-chamber cross section or the apical two-chamber cross section is set as the optimal measurement cross section, the feature value calculation unit 24 can calculate, as a feature value, the similarity between a plurality of frames of ultrasound images U and a reference image corresponding to the apical four-chamber cross section or the apical two-chamber cross section set as the optimal measurement cross section. The feature value calculation unit 24 can calculate the similarity, for example, by a so-called template matching method, a method using a trained model in machine learning that has learned a relationship between similarities between a large number of ultrasound images U and a reference image, or the like.

In this case, the probe operation direction calculation unit 27 can calculate the current rotation direction of the ultrasound probe 1 by the user as the target operation direction in a case in which a plurality of similarities calculated by the feature value calculation unit 24 change to gradually increase in time series from any time point in the past to the present. In addition, the probe operation direction calculation unit 27 can calculate the opposite direction to the current rotation direction of the ultrasound probe 1 by the user as the target operation direction in a case in which a plurality of similarities calculated by the feature value calculation unit 24 change to gradually decrease in time series from any time point in the past to the present. Accordingly, the user can easily adjust the rotation direction to acquire an ultrasound image U that appropriately represents the apical four-chamber cross section or the apical two-chamber cross section.

In general, in a case of calculating the left ventricular ejection fraction of heart H, the major axis diameter L and the minor axis diameter of the lumen of the left ventricle A at end diastole and end systole in the apical four-chamber cross section and the major axis diameter L and the minor axis diameter of the lumen of left ventricle A at end diastole and end systole in the apical two-chamber cross section are measured, and the left ventricular ejection fraction is often calculated using the major axis diameter L and the minor axis diameter of the lumen of the left ventricle A in the apical four-chamber cross section and the major axis diameter L and the minor axis diameter of the lumen of the left ventricle A in the apical two-chamber cross section. Here, the minor axis diameter of the lumen of the left ventricle A refers to the length of the minor axis defined by a line segment that is orthogonal to the major axis B of the lumen of the left ventricle A and has the maximum length.

In a case of imaging both the apical four-chamber cross section and the apical two-chamber cross section, by applying the present invention to the imaging of both the apical four-chamber cross section and the apical two-chamber cross section, a message M or the like indicating the target operation direction for the apical four-chamber cross section and the apical two-chamber cross section is displayed on the monitor 23, as shown in, for example, Figs. 4, 7, and 10. Therefore, an ultrasound image U representing the optimal measurement cross section corresponding to the apical four-chamber cross section and an ultrasound image U representing the optimal measurement cross section corresponding to the apical two-chamber cross section can be easily acquired.

In this case, the image acquisition unit 31 can acquire a plurality of frames of ultrasound images U in order for each of the apical four-chamber cross section and the apical two-chamber cross section of the heart H. Since the value of the optimal feature value is expected to be the same for the apical four-chamber cross section and the apical two-chamber cross section, the feature value calculation unit 24 can calculate a plurality of feature values for a first cross section among the apical four-chamber cross section and the apical two-chamber cross section for which the plurality of frames of ultrasound images U have been acquired first by the image acquisition unit 31, the optimal feature value selection unit 25 can select the optimal feature value from the plurality of feature values calculated for the first cross section, and the notification unit 28 can notify the user of the optimal feature value selected for the first cross section while the plurality of frames of ultrasound images U for a second cross section among the apical four-chamber cross section and the apical two-chamber cross section that is different from the first cross section are being acquired by the image acquisition unit 31. Accordingly, the process of selecting and storing optimal feature values for a second cross section that is imaged after the first cross section is omitted, thereby reducing the calculation load required for processing by the processor 32.

Although an example in which the feature value calculation unit 24 calculates the feature value related to the left ventricle A has been described, the target for calculating the feature value is not limited to the left ventricle A. For example, in a case of measuring the right ventricular ejection fraction of the heart H, the feature value calculation unit 24 can calculate feature values related to the right ventricle, such as the depth of the upper end part of the lumen of the right ventricle, the major axis diameter of the lumen of the right ventricle, or the area of the lumen of the right ventricle. In addition, depending on the type of measurement, the feature value calculation unit 24 can also calculate feature values related to the left atrium or the right atrium. In this manner, the feature value calculation unit 24 can calculate the feature values related to any one of the left ventricle A, the right ventricle, the left atrium, and the right atrium, that is, the cardiac cavity.

Further, although an example in which the measurement target organ is the heart H has been described, the measurement target organ is not limited to the heart H, and for example, the bladder of the subject can be set as the measurement target organ. In this case, the feature value calculation unit 24 can calculate the depth of the shallowest part of the contour of the bladder in the ultrasound image U and the major axis diameter of the bladder in the ultrasound image U as the feature values. The major axis diameter of the bladder in the ultrasound image U is calculated from the distance of the longest line segment among distances between two points on the contour of the bladder.

Similarly to the case in which the measurement target organ is the heart H, the probe operation direction calculation unit 27 can calculate the current movement direction of the ultrasound probe 1 as the target operation direction in a case in which the depth value of the shallowest part of the contour of the bladder decreases in time series. Also, similarly to the case in which the measurement target organ is the heart H, the probe operation direction calculation unit 27 can calculate the current inclination direction of the ultrasound probe 1 as the target operation direction in a case in which the value of the major axis diameter of the bladder decreases in time series.

Further, the measurement item or the type of optimal measurement cross section can be input by the user via the input device 30 before the ultrasound image U is acquired. In this case, the feature value calculation unit 24 calculates, from the ultrasound image U, the feature value corresponding to the input measurement item or type of the optimal measurement cross section.

In addition, for example, the main body controller 29 can also estimate the measurement item or the type of the optimal measurement cross section by analyzing the acquired ultrasound image U. The main body controller 29 can estimate the measurement item or the type of optimal measurement cross section from the ultrasound image U, for example, by using a trained model in machine learning that has learned a relationship between a large number of ultrasound images U and parts of the subject, such as the left ventricle A, the right ventricle, or the bladder, and a relationship between parts of the subject and the measurement item or the type of optimal measurement cross section. In this case, the feature value calculation unit 24 calculates, from the ultrasound image U, the feature value corresponding to the estimated measurement item or type of the optimal measurement cross section.

The ultrasound diagnostic apparatus may also comprise a position and posture sensor for detecting the position and inclined angle of the ultrasound probe 1. The position and posture sensor can include, for example, at least one of a so-called inertial sensor, a magnetic sensor, an optical sensor, or an optical camera. The inertial sensor can include, for example, at least one of a so-called acceleration sensor or a gyro sensor. The position and posture sensor may be incorporated in the ultrasound probe 1 or attached to a housing of the ultrasound probe 1. In addition, in a case in which a sensor device that measures the ultrasound probe 1 from the outside, such as an optical sensor, is used as the position and posture sensor, the position and posture sensor may be disposed at a position away from the ultrasound probe 1. In addition, the position and posture sensor can also be composed of a marker, such as a so-called augmented reality (AR) marker, attached to the ultrasound probe 1, an optical camera that captures an optical image of the marker, and a marker recognition unit that recognizes the marker in the optical image and calculates the position and the inclined angle of the ultrasound probe 1 from the size and inclined angle of the marker, and the like. The marker recognition unit can be incorporated in the processor 32, for example.

In a case in which the ultrasound diagnostic apparatus comprises a position and posture sensor, the probe operation direction calculation unit 27 can calculate the target operation direction by further taking into account the position and the inclined angle of the ultrasound probe 1 detected by the position and posture sensor. For example, in a case in which the ultrasound probe 1 is moved in parallel along the body surface of the subject and a case in which the ultrasound probe 1 is inclined, both the depth D of the upper end part E of the lumen of the left ventricle A and the major axis diameter L of the lumen of the left ventricle A change. However, by referring to the detection results of the position and posture sensor, the probe operation direction calculation unit 27 can reliably determine whether the ultrasound probe 1 is moving in parallel along the body surface of the subject or tilting relative to the body surface, and can accurately calculate the target operation direction.

### Explanation of References

1: ultrasound probe
2: apparatus main body
11: transducer array
12: transmission/reception circuit
21: image generation unit
22: display controller
23: monitor
24: feature value calculation unit
25: optimal feature value selection unit
26: feature value memory
27: probe operation direction calculation unit
28: notification unit
29: main body controller
30: input device
31: image acquisition unit
32: processor
41: pulser
42: amplification unit
43: AD conversion unit
44: beam former
45: signal processing unit
46: DSC
47: image processing unit
A: left ventricle
B: major axis
C: apex of heart
D, D1, D2, D3: depth
E: upper end part
G1: depth indication line
G2: major axis indication line
H: heart
L, L1, L2, L3: major axis diameter
M: message
P1, P2: scan plane
U: ultrasound image

## Claims

1. An ultrasound diagnostic apparatus comprising:
an ultrasound probe (1);
an image acquisition unit (31) that acquires a plurality of frames of ultrasound images in which a measurement target organ of a subject is imaged by transmitting and receiving an ultrasound beam using the ultrasound probe (1);
a feature value calculation unit (24) that calculates a feature value of the measurement target organ in each of the plurality of frames of ultrasound images by performing image analysis on the plurality of frames of ultrasound images acquired by the image acquisition unit (31);
a probe operation direction calculation unit (27) that calculates a target operation direction of the ultrasound probe (1) for imaging an optimal measurement cross section suitable for measuring the measurement target organ based on a time series change in a plurality of the feature values calculated by the feature value calculation unit (24) in the plurality of frames of ultrasound images; and
a notification unit (28) that notifies a user of the target operation direction calculated by the probe operation direction calculation unit (27).

2. The ultrasound diagnostic apparatus according to claim 1,
wherein the probe operation direction calculation unit (27) starts calculating the target operation direction with a trigger of an elapse of a predetermined time after the image acquisition unit (31) starts acquiring the ultrasound image.

3. The ultrasound diagnostic apparatus according to either claim 1 or claim 2, further comprising:
an optimal feature value selection unit (25) that selects one of the plurality of feature values calculated by the feature value calculation unit (24) as an optimal feature value corresponding to the optimal measurement cross section,
wherein the notification unit (28) notifies the user of the optimal feature value selected by the optimal feature value selection unit (25).

4. The ultrasound diagnostic apparatus according to any one of claims 1 to 3,
wherein the notification unit (28) notifies the user to acquire the plurality of frames of ultrasound images while moving the ultrasound probe (1).

5. The ultrasound diagnostic apparatus according to any one of claims 1 to 4, further comprising:
a feature value memory (26) that stores the optimal feature value in association with the subject.

6. The ultrasound diagnostic apparatus according to any one of claims 1 to 5,
wherein the measurement target organ is a heart.

7. The ultrasound diagnostic apparatus according to claim 6,
wherein the feature value calculation unit (24) calculates a depth of an upper end part of a cardiac cavity in the plurality of frames of ultrasound images as the feature value, and
the probe operation direction calculation unit (27)
calculates a current movement direction of the ultrasound probe (1) by the user along a body surface of the subject as the target operation direction in a case in which the depth calculated by the feature value calculation unit (24) changes to become shallower in time series, and
calculates an opposite direction to the current movement direction of the ultrasound probe (1) by the user along the body surface of the subject as the target operation direction in a case in which the depth calculated by the feature value calculation unit (24) changes to become deeper in time series.

8. The ultrasound diagnostic apparatus according to claim 6 or 7,
wherein the feature value calculation unit (24) calculates a length of a major axis of a cardiac cavity as the feature value, and
the probe operation direction calculation unit (27)
calculates a current inclination direction of the ultrasound probe (1) by the user as the target operation direction in a case in which the length calculated by the feature value calculation unit (24) changes to become longer in time series, and
calculates an opposite direction to the current inclination direction of the ultrasound probe (1) by the user as the target operation direction in a case in which the length calculated by the feature value calculation unit (24) changes to become shorter in time series.

9. The ultrasound diagnostic apparatus according to any one of claims 6 to 8,
wherein the feature value calculation unit (24) calculates an area of a cardiac cavity as the feature value, and
the probe operation direction calculation unit (27)
calculates a current inclination direction of the ultrasound probe (1) by the user as the target operation direction in a case in which the area calculated by the feature value calculation unit (24) changes to become larger in time series, and
calculates an opposite direction to the current inclination direction of the ultrasound probe (1) by the user as the target operation direction in a case in which the area calculated by the feature value calculation unit (24) changes to become smaller in time series.

10. The ultrasound diagnostic apparatus according to any one of claims 6 to 9,
wherein the plurality of feature values change in time series due to pulsation of the heart to have a plurality of maximum values and a plurality of minimum values, and
the probe operation direction calculation unit (27) calculates the target operation direction based on a time series change in the plurality of maximum values or the plurality of minimum values.

11. The ultrasound diagnostic apparatus according to any one of claims 6 to 10,
wherein the image acquisition unit (31) acquires the plurality of frames of ultrasound images in order for each of an apical four-chamber cross section and an apical two-chamber cross section of the heart,
the feature value calculation unit (24) calculates the plurality of feature values for a first cross section among the apical four-chamber cross section and the apical two-chamber cross section for which the plurality of frames of ultrasound images have been acquired first by the image acquisition unit (31),
the optimal feature value selection unit (25) selects the optimal feature value from the plurality of feature values calculated for the first cross section, and
the notification unit (28) notifies the user of the optimal feature value selected for the first cross section while the plurality of frames of ultrasound images for a second cross section among the apical four-chamber cross section and the apical two-chamber cross section that is different from the first cross section are being acquired by the image acquisition unit.

12. The ultrasound diagnostic apparatus according to any one of claims 6 to 11,
wherein the feature value calculation unit (24) calculates a similarity between the plurality of frames of ultrasound images and a reference image corresponding to the optimal measurement cross section as the feature value, and
the probe operation direction calculation unit (27)
calculates a current rotation direction of the ultrasound probe (1) by the user as the target operation direction in a case in which a plurality of the similarities calculated by the feature value calculation unit (24) change to become larger in time series, and
calculates an opposite direction to the current rotation direction of the ultrasound probe (1) by the user as the target operation direction in a case in which the plurality of similarities calculated by the feature value calculation unit (24) change to become smaller in time series.

13. The ultrasound diagnostic apparatus according to any one of claims 1 to 12, further comprising:
a position and posture sensor that detects a position and an inclined angle of the ultrasound probe (1),
wherein the probe operation direction calculation unit (27) calculates the target operation direction by further taking into account the position and the inclined angle of the ultrasound probe (1) detected by the position and posture sensor.

14. The ultrasound diagnostic apparatus according to any one of claims 1 to 13, further comprising:
a monitor (23),
wherein the notification unit (28) notifies the user of the target operation direction by displaying the target operation direction on the monitor (23).

15. A control method for an ultrasound diagnostic apparatus, the control method comprising:
acquiring a plurality of frames of ultrasound images in which a measurement target organ of a subject is imaged by transmitting and receiving an ultrasound beam using an ultrasound probe (1);
calculating a feature value of the measurement target organ in each of the plurality of frames of ultrasound images by performing image analysis on the plurality of frames of ultrasound images that have been acquired;
calculating a target operation direction of the ultrasound probe (1) for imaging an optimal measurement cross section suitable for measuring the measurement target organ based on a time series change in a plurality of the feature values that have been calculated in the plurality of frames of ultrasound images; and
notifying a user of the calculated target operation direction.
